# EUROPÄISCHE PATENTSCHRIFT

(11) **EP 0 852 469 B1**
(45) Veröffentlichungstag und Bekanntmachung des Hinweises auf die Patenterteilung: **21.11.2001**
(21) Anmeldenummer: 96932564.6
(22) Anmeldetag: 18.09.1996
(51) Int. Cl.: A23L 1/305, A61K 31/195

(54) **VERWENDUNG VON THREONIN ZUR BEHANDLUNG DER PHENYLKETONURIE**
USE OF THREONINE FOR THE TREATMENT OF PHENYLKETONURIA
UTILISATION DE LA THREONINE POUR LE TRAITEMENT DE LA PHENYLCETONURIE

(30) Priorität: 18.09.1995 DE 19534602
(43) Veröffentlichungstag der Anmeldung: 15.07.1998
(73) Patentinhaber: N.V. Nutricia, 2712 HM Zoetermeer (NL)
(72) Erfinder: GEORGI, Gilda, D-61381 Friedrichsdorf (DE); SAWATZKI, Günther, D-35516 Münzenberg (DE)
(74) Vertreter: Köster, Hajo, Dr.
(86) Internationale Anmeldenummer: EP9604093
(87) Internationale Veröffentlichungsnummer: WO9712527

(56) Entgegenhaltungen:
- EP-A- 0 488 078
- EP-A- 0 492 183
- DD-A- 294 865
- DE-A- 2 654 820
- JP-A- 59 005 111

## Beschreibung

Die Erfindung betrifft ein Mittel zur diätetischen oder oralen pharmazeutischen Behandlung der Phenylketonurie sowie die Verwendung von Threonin zur Herstellung eines diätetischen oder oralen pharmazeutischen Mittels zur Behandlung der Phenylketonurie.

Die Phenylketonurie (nachstehend PKU genannt) gehört zu den genetisch bedingten Erkrankungen und stellt eine Stoffwechselstörung dar, die unbehandelt meist zu einer schweren körperlichen und geistigen Entwicklungsstörung führt. Phenylalanin wird unter normalen Ernährungsbedingungen mit allen Proteinen tierischer und pflanzlicher Herkunft aufgenommen. Auf Grund der Stoffwechselstörung häuft sich bei den erkrankten Personen das Phenylalanin im Blut und Geweben an. Die Folge des teilweise exzessiv erhöhten Phenylalaninspiegels sind Stoffwechselimbalanzen, die über verschiedene Symptome bis zur irreversiblen, fortschreitenden geistigen Behinderung führen können.

Die PKU ist nicht heilbar, kann aber durch entsprechende Diäten behandelt werden, die phenylalaninarm sind, so daß über die Nahrung nur eine solche Menge Phenylalanin zugeführt wird, daß die Synthese körpereigener Proteine für Wachstum und Regenerierung optimal gewährleistet ist und gleichzeitig der Phenylalaninblutspiegel im Normbereich liegt.

Da alle Proteine Phenylalanin enthalten, müssen diese Diäten extrem eiweißarm sein. Daher wird den PKU-Patienten mit einer phenylalaninarmen Ernährung zu wenig von allen anderen, für das Leben ebenso wichtigen Aminosäuren zugeführt. Deshalb werden die übrigen Aminosäuren zusätzlich in Form eines Diätetikums zugeführt, welches meistens freie Aminosäuren enthält. Ein derartiges Diätetikum ist beispielsweise in der EP-A 0 488 078 beschrieben. Eine phenylalaninfreie Säuglings- und Kleinkindernahrungsgrundlage ist ferner in der EP-A 0 492 183 beschrieben. Dort finden sich auch weitere Ausführungen bezüglich der hier in Rede stehenden PKU-Problematik. Ferner sind dort auch weitere diätetische Spezialerzeugnisse zur Behandlung der PKU erläutert. Auf die Offenbarung dieser europäischen Patentanmeldungen wird hiermit ausdrücklich Bezug genommen.

Der Hauptnachteil der bisher bekannten phenylalaninfreien Aminosäuremischungen und der darauf basierenden Diätetika liegt in ihrem äußerst schlechten Geschmack, so daß derartige phenylalaninfreie Diätetika häufig von den Patienten, welche diese zweckmäßigerweise lebenslang einnehmen müssen, abgelehnt werden.

Aufgabe der vorliegenden Erfindung ist es, einen verbesserten und einfacheren Weg zur Ernährungsbehandlung oder pharmazeutischen Behandlung der Phenylketonurie bereitzustellen.

Gelöst wird diese Aufgabe durch die Lehre der unabhängigen Ansprüche.

Es wurde überraschend gefunden, daß durch zusätzliche und insbesondere hohe Gaben von Threonin die Konzentration von Phenylalanin im Plasma von PKU-Patienten gesenkt werden kann. Durch die orale Gabe von Threonin, welche zu hohen Threonin-Blutspiegeln führt, kann der Phenylalaninspiegel im Blut gesenkt werden. Dies hat beispielsweise zur Folge, daß bei zusätzlicher Applikation von Threonin die Nahrung von PKU-Patienten hinsichtlich des Phenylalaningehaltes weniger stark kontrolliert werden muß. Zudem kann durch die phenylalaninsenkende Wirkung des Threonins dem PKU-Patienten mehr Phenylalanin und somit auch mehr Protein über die Nahrung zugeführt werden, ohne daß es zu gesundheitlichen Beeinträchtigungen kommt. Dies hat den großen Vorteil, daß deutlich mehr Nahrungsmittel für den PKU-Patienten geeignet sind. Gleichzeitig kann die bisher notwendige tägliche Zufuhr an freien Aminosäuren entsprechend reduziert werden.

Wenn im Rahmen der vorliegenden Unterlagen von dem Begriff "zusätzlich" im Zusammenhang mit der Menge an Threonin die Rede ist, dann heißt dies, daß einem Patienten eine Menge an Threonin zugeführt oder verabreicht wird, die höher bzw. größer ist als diejenige Menge, die ein PKU-Patient bisher zu sich nahm, beispielsweise mit der üblichen Nahrung einschließlich üblicher PKU-Zusätze, bzw. die ein PKU-Patient auf Grund bisheriger Empfehlungen zu sich nehmen sollte.

Das zusätzliche Threonin kann einem PKU-Patienten in jeder geeigneten Form verabreicht werden. So ist es beispielsweise möglich, gewöhnliche Lebensmittel mit Threonin zu versetzen und dadurch dem Patienten eine erhöhte Threoninmenge zuzuführen. Ferner ist es möglich, die erhöhte Threoningabe in Form einer Nahrung und insbesondere einer Formelnahrung zuzuführen. Auch die Verabreichung in Form eines Pharmazeutikums ist möglich.

Zu derartigen Nahrungen etc. zählen beispielsweise Säuglingsmilchen, Kindernahrungen, Nahrungen für Jugendliche und Erwachsene, Diätetika und Nahrungszusätze.

Der Kern der vorliegenden Erfindung besteht somit darin, daß man einem PKU-Patienten zusätzliche Threoninmengen verabreicht, um auf diese Weise den Phenylalaninblutspiegel zu senken. Die Art der Threoninverabreichung ist dabei von untergeordneter Bedeutung und sollte entsprechend den Bedürfnissen des Patienten, den Anforderungen des Diätplanes oder unter Berücksichtigung weiterer, für den Patienten wichtiger Kriterien gewählt werden.

Entscheidend ist somit lediglich, daß ein PKU-Patient eine zusätzliche Threoninmenge auf beliebige Art und Weise zu sich nimmt. Erfindungsgemäß nimmt ein PKU-Patient mindestens eine solche Threoninmenge zu sich, daß die dem Körper zugeführte Threoninmenge mindestens 15 g pro 100 g der insgesamt dem Körper zugeführten Aminosäurenmenge ausmacht. Wenn im Rahmen der vorliegenden Anmeldung von einem Threoninmengenbereich von 15 g bis 100 g oder von 15 g bis 80 g die Rede ist, dann sind damit alle dazwischen liegenden Einzelwerte offenbart, beispielsweise 16, ... 30, 31, ... 45, 46, 47, 48, 49, 50, ... 60, 61 ..., 70, 71, 72, ..., 80, 81, ..., 90, 91, ... 100.

Das Threonin kann den einzelnen Lebensmitteln, Nahrungszusätzen, Zubereitungen und pharmazeutischen Mitteln in Form der freien Aminosäure einverleibt sein. Zudem kann das Threonin in Form seiner Salze, in Form von threoninhaltigen Peptiden (beispielsweise bovines Glycomakropeptid), in Form von threoninreichen Proteinen und deren Hydrolysaten sowie in Form von Mischungen dieser Threoninquellen vorliegen.

Analoges gilt für die anderen Aminosäuren, die in jeder geeigneten Form, beispielsweise als Peptide, als Proteine und als freie Aminosäuren oder als Mischung davon, vorliegen können.

Im einfachsten Falle kann somit ein PKU-Patient eine übliche Nahrung zu sich nehmen. Um nun auch die erforderliche Menge an Threonin einzunehmen, kann dieser Patient das Threonin als freie Säure, die beispielsweise einer Kapsel einverleibt ist, zu sich nehmen.

Das Haupteinsatzgebiet der Erfindung liegt darin, einen Teil der bereits bekannten und auf dem Markt vorhandenen, im allgemeinen sehr schlecht schmeckenden PKU-Nahrungen durch threoninreiche Produkte oder Threonin in freier Form ersetzen zu können. Durch dieses Threonin werden die Phenylalaninspiegel im Blut der Patienten gesenkt, so daß vermehrt auch herkömmliche Nahrungsmittel, wie Säuglingsmilchen, Breie, Milch, Milchprodukte, Obst, Gemüse usw. für die Diäten verwendet werden können.

Gegenstand der Erfindung ist auch ein Mittel in Form eines diätetischen Lebensmittels oder einer Nahrungsgrundlage, die das Threonin in einer Menge von 45 bis 80 g pro 100 g Aminosäuren enthält. Die Threoninmenge wird dabei in Abhängigkeit davon gewählt, welche weiteren Lebensmittel und/oder Nahrungsmittelzusätze, beispielsweise bisher bekannte Nahrungsmittelzusätze, dem Patienten zur Verfügung gestellt werden.

Gegenstand der Erfindung ist ferner ein Mittel in Form eines oralen pharmazeutischen Mittels oder eines Nahrungsmittelzusatzes, in dem das Threonin 45 bis 100 g pro 100 g Aminosäuren ausmacht. Im einfachsten Fall kann man somit einem Patienten ein pharmazeutisches Mittel oder einen Nahrungsmittelzusatz zur Verfügung stellen, das bzw. der als wirksamen Bestandteil ausschließlich Threonin enthält. So ist es beispielsweise möglich, übliche Kapseln mit reinem Threonin (freie Aminosäure) zu verabreichen. Durch Gabe von derartigem freien Threonin bzw. Threonin-Kapseln kann der Threoningehalt von Nahrungen, Nahrungszusätzen und PKU-Diätetika angehoben werden, ohne die anderen Aminosäuren und/oder Proteine in ihrer Zusammensetzung zu ändern.

Wenn im Rahmen der vorliegenden Unterlagen von einer bestimmten Menge Threonin pro 100 g Aminosäuren die Rede ist, dann gehen bei den Berechnungen die Werte für die freien Aminosäuren ein, auch wenn diese Aminosäuren in gebundener Form und somit als Peptid oder Protein vorliegen. Die für das Threonin und für die Aminosäuren aufgeführten Mengenangaben beziehen sich somit auf die freien Säuren.

Die Erfindung wird im folgenden anhand der nachstehenden Beispiele näher erläutert.

### Beispiel 1: Säuglingsmilch für Kinder von 0 bis 3 Monaten (Tagesmenge)

Es wird von einer für dieses Alter üblichen PKU-Diät ausgegangen, beispielsweise der Zusammensetzung Milupa PKU 1 für Säuglinge. 100 g PKU 1 enthalten 60,3 g Aminosäuren (gesamt; entspricht in etwa 50,3 g Eiweiß), 0,0 g Fett, 21,0 g Kohlenhydrate und 15,8 Mineralstoffe und Spurenelemente, Rest Wasser.

50 % dieser Zusammensetzung werden durch eine dem Proteingehalt entsprechende Menge an Glykomakropeptid und/oder einer handelsüblichen Säuglingsmilchnahrung, (beispielsweise Milumil 1) ersetzt. Es werden somit eine solche Menge an Glykomakropeptid und/oder einer handelsüblichen Säuglingsmilchnahrung zugegeben, daß der Proteingehalt dieser zugegebenen Menge an Glykomakropeptid und/oder an einer handelsüblichen Säuglingsmilchnahrung dem Proteingehalt des ersetzten Teils der PKU-Diät entspricht. Dazu werden die verschiedenen Proteinquellen derart vermischt, daß 2,5 bis 3,8 g Protein aus der PKU-Diät, 2,5 bis 2,8 g Protein aus dem Glykomakropeptid und 3,8 bis 4,2 g Protein aus der handelsüblichen Säuglingsmilchnahrung stammen. Die in dieser Mischung enthaltene Threoninmenge beträgt 1,0 bis 1,2 g und liegt um den Faktor 3,3 bis 2,4 höher als die für gesunde Kinder empfohlene Mindestmenge an Threonin.

### Beispiel 2: Diät für Kinder von 1 bis 3 Jahren (Tagesmenge)

Es wird von einer für dieses Alter üblichen PKU-Diät ausgegangen, beispielsweise der Zusammensetzung Milupa PKU 2 für Klein- und Schulkinder. 100 g PKU 2 enthalten 80,1 g Aminosäuren, (gesamt; entspricht 66,8 g Eiweiß), 0,0 g Fett, 8,5 g Kohlenhydrate und 7,7 g Mineralstoffe und Spurenelemente (Rest Wasser). 25 % dieser phenylalaninfreien PKU-Diät werden durch eine dem Proteingehalt entsprechende Menge an handelsüblichen Fertigbreien, Obst und Gemüse ersetzt. Die Proteinquellen werden dazu derart gemischt, daß 10,1 g Protein der fertigen Diät aus der üblichen PKU-Diät und 6,0 g Protein aus Fertigbreien, Obst und/öder Gemüse stammen.

Gleichzeitig werden den diese Diät einnehmenden Kindern 4 g Threonin in freier Form pro Tag in Form von Kapseln verabreicht.

Die in dieser Kombination aus Diät und Threonin in Kapselform enthaltende Menge an Threonin beträgt 4,9 g und liegt um den Faktor 5,4 höher als die für gesunde Kinder empfohlene tägliche Mindestmenge an Threonin.

### Beispiel 3: Diät für Schulkinder von 7 bis 10 Jahren (Tagesmenge)

Man geht aus von der im Beispiel 2 beschriebenen PKU-Diät. 70 % dieser phenylalaninfreien PKU-Diät werden durch eine dem Proteingehalt entsprechende Mischung an Glykomakropeptid und handelsüblichen Milchprodukten, Obst, Gemüse usw. ersetzt. Dazu werden die Proteinquellen derart vermischt, daß 6,7 g Protein der fertigen Diät aus der am Anfang eingesetzten PKU-Diät, 12,3 g Protein aus dem Glykomakropeptid und 8,2 g Protein aus den handelsüblichen Milchprodukten, Obst, Gemüse usw. stammt.

Gleichzeitig werden den behandelten Schulkindern 7 g Threonin verabreicht, die in Form von Kapseln oder Dragees vorliegen können oder einem Instant-Getränk einverleibt sind.

Die in dieser Kombination den Schulkindern insgesamt verabreichte Threoninmenge beträgt 10,1 g und liegt um den Faktor 10,1 höher als die für gesunde Kinder empfohlene tägliche Mindestmenge an Threonin.

## Patentansprüche

1. Verwendung von Threonin zur Herstellung eines diätetischen oder oralen pharmazeutischen Mittels zur Behandlung der Phenylketonurie bzw. zur Absenkung des Phenylalaninspiegels im Plasma, wobei das Threonin in einer Menge von mindestens 15 g pro 100 g Aminosäuren eingesetzt wird.

2. Verwendung von Threonin nach Anspruch 1, wobei das diätetische oder orale pharmazeutische Mittels das Threonin als freie Aminosäure, als Salz, als threoninhaltiges Peptid, als threoninreiches Protein oder als Proteinhydrolysat oder als Mischung davon enthält.

3. Verwendung von Threonin nach Anspruch 1 oder 2, wobei das diätetische oder orale pharmazeutische Mittel als Wirkstoff ausschließlich Threonin enthält.

4. Verwendung von Threonin nach Anspruch 3, wobei das diätetische oder orale pharmazeutische Mittel als Wirkstoff ausschließlich Threonin als freie Aminosäure enthält.

5. Mittel zur diätetischen oder oralen pharmazeutischen Behandlung der Phenylketonurie,
**gekennzeichnet durch**
einen Gehalt an mindestens 45 g Threonin pro 100 g Aminosäuren.

6. Mittel nach Anspruch 5,
**dadurch gekennzeichnet,**
**daß** das Threonin als freie Aminosäure, als Salz, als threoninhaltiges Peptid, als threoninreiches Protein oder als Proteinhydrolysat oder als Mischung davon vorliegt.

7. Mittel nach Anspruch 6,
**dadurch gekennzeichnet,**
**daß** es als threoninhaltiges Peptid bovines Glykomakropeptid enthält.

8. Mittel nach einem der Ansprüche 5 bis 7 in Form eines diätetischen Lebensmittels, einer PKU-Diät oder einer Nahrungsgrundlage,
**dadurch gekennzeichnet,**
**daß** es das Threonin in einer Menge von 45 bis 80 g pro 100 g Aminosäuren enthält.

9. Mittel nach einem der Ansprüche 5 bis 7 in Form eines oralen pharmazeutischen Mittels oder eines Nahrungsmittelzusatzes,
**dadurch gekennzeichnet,**
**daß** es das Threonin in einer Menge von 45 bis 100 g pro 100 g Aminosäuren enthält.

10. Mittel nach Anspruch 9,
**dadurch gekennzeichnet,**
**daß** es als Wirkstoff ausschließlich Threonin enthält.

11. Mittel nach Anspruch 10,
**dadurch gekennzeichnet,**
**daß** es als Wirkstoff ausschließlich Threonin als freie Aminosäure enthält.

## Claims

1. Use of threonine for the production of a dietetic or oral pharmaceutical composition for the treatment of phenylketonuria or for lowering the phenyl-alanine level in the plasma whereby the threonine is used in an amount of at least 15 g per 100 g amino acids.

2. Use of threonine according to claim 1 whereby the dietetic or oral pharmaceutical composition contains the threonine as free amino acid, as salt, as threonine-containing peptide, as threonine-rich protein or as protein hydrolysate or as a mixture thereof.

3. Use of threonine according to claim 1 or 2 whereby the dietetic or oral pharmaceutical composition contains as active component exclusively threonine.

4. Use of threonine according to claim 3 whereby the dietetic or oral pharmaceutical composition contains as active component exclusively threonine as free amino acid.

5. Composition for the dietetic or oral pharmaceutical treatment of phenylketonuria,
**characterized by** a content of at least 45 g threonine per 100 g amino acids.

6. Composition according to claim 5,
**characterized in that**
the threonine is present as free amino acid, as salt, as threonine-containing peptide, as threonine-rich protein or as protein hydrolysate or as a mixture thereof.

7. Composition according to Claim 6,
**characterized in that**
it contains bovine glycomacropeptide as threonine-containing peptide.

8. Composition according to one of the claims 5 to 7 in the form of a dietetic food, of a PKU diet, or a food base,
**characterized in that**
it contains threonine in an amount of 45 to 80 g per 100 g amino acids.

9. Composition according to one of the claims 5 to 7 in the form of an oral pharmaceutical composition or a food supplement,
**characterized in that**
it contains threonine in an amount of 45 to 100 g per 100 g amino acids.

10. Composition according to Claim 9,
**characterized in that**
as active component it contains exclusively threonine.

11. Composition according to Claim 10,
**characterized in that**
as active component it contains exclusively threonine as free amino acid.

## Revendications

1. Utilisation de thréonine pour préparer un agent diététique ou pharmaceutique oral destiné au traitement de la phénylcétonurie ou destiné à réduire le taux de phénylalanine dans le plasma, la thréonine étant utilisée dans une quantité au moins égale à 15 g pour 100 g d'acides aminés.

2. Utilisation de thréonine selon la revendication 1, dans laquelle l'agent diététique ou pharmaceutique oral contient la thréonine en tant qu'acide aminé libre, en tant que sel, en tant que peptide contenant de la thréonine, en tant que protéine riche en thréonine ou en tant qu'hydrolysat de protéine, ou en tant que mélange de ceux-ci.

3. Utilisation de thréonine selon la revendication 1 ou 2, dans laquelle l'agent diététique ou pharmaceutique oral contient exclusivement de la thréonine en tant que substance active.

4. Utilisation de thréonine selon la revendication 3, dans laquelle l'agent diététique ou pharmaceutique oral contient exclusivement de la thréonine sous forme d'acide aminé libre en tant que substance active.

5. Agent pour le traitement diététique ou pharmaceutique oral de la phénylcétonurie, **caractérisé par** une teneur au moins égale à 45 g de thréonine pour 100 g d'acides aminés.

6. Agent selon la revendication 5, **caractérisé en ce que** la thréonine se présente sous la forme d'acides aminés libres, sous la forme d'un sel, sous la forme d'un peptide contenant de la thréonine, sous la forme d'une protéine riche en thréonine ou sous la forme d'un hydrolysat de protéine, ou en tant que mélange de ceux-ci.

7. Agent selon la revendication 6, **caractérisé en ce qu'**il contient en tant que peptide contenant de la thréonine un glycomacropeptide bovin.

8. Agent selon l'une des revendications 5 à 7 sous la forme d'une nourriture diététique, d'un régime PCU ou d'une base alimentaire, **caractérisé en ce qu'**il contient la thréonine dans une quantité comprise entre 45 et 80 g pour 100 g d'acides aminés.

9. Agent selon l'une des revendications 5 à 7 sous la forme d'un agent pharmaceutique oral ou d'un complément alimentaire, **caractérisé en ce qu'**il contient la thréonine dans une quantité comprise entre 45 et 100 g pour 100 g d'acides aminés.

10. Agent selon la revendication 9, **caractérisé en ce qu'**il contient exclusivement de la thréonine en tant que substance active.

11. Agent selon la revendication 10, **caractérisé en ce qu'**il contient exclusivement de la thréonine sous forme d'acides aminés libres en tant que substance active.
